# EUROPEAN PATENT APPLICATION

(11) **EP 1 541 198 A1**
(43) Date of publication of application: **15.06.2005**
(21) Application number: 03028137.2
(22) Date of filing: 05.12.2003
(51) Int. Cl.: A61P 35/00, A61P 37/02, A61P 29/00, A61K 31/194, C07C 233/60

(54) **Cycloalkyl compounds as anti-inflammatory, immunomodulatory and anti-proliferatory agents**

(71) Applicant: 4SC AG, 82152 Martinsried (DE)
(72) Inventor: Leban, Johann, Dr., 82110 Germering (DE)
(74) Representative: Strehl Schübel-Hopf & Partner

(57) **Abstract**

The present invention relates to compounds of the general formula (I) 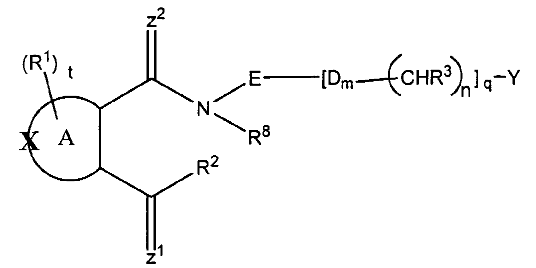 or salts or isomeres thereof, wherein A is a 4-8 membered non-aromatic ring system,
and to compounds of the general formula (II) 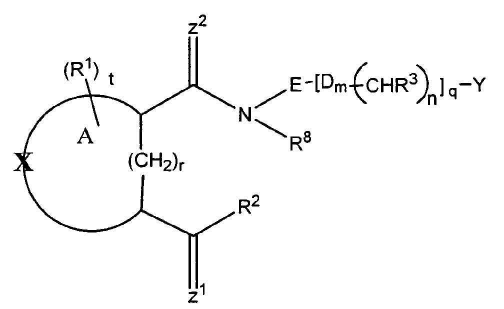 or salts or isomeres thereof, wherein A is a 3-8 membered non-aromatic ring system, wherein, when r = 0, there is no double bond between the carbon atoms carrying the substituents -CZ¹- and -CZ²-;

## Description

The present invention relates to novel compounds that can be used as antiinflammatory, immunomodulatory and antiproliferatory agents. In particular the invention refers to new compounds which inhibit dihydroorotate dehydrogenase (DHODH), a process for their manufacture, pharmaceutical compositions containing them and to their use for the treatment and prevention of diseases, in particular their use in diseases where there is an advantage in inhibiting dihydroorotate dehydrogenase (DHODH).

Rheumatoid arthritis (RA) is a disease which is quite common especially among elder people. Its treatment with usual medications as for example non-steroid anti-inflammatory agents is not satisfactory. In view of the increasing ageing of the population, especially in the developed Western countries or in Japan the development of new medications for the treatment of RA is urgently required.

WO 99/38846 and EP 0 646 578 disclose compounds which are reported to be useful for treatment of RA.

A medicament against rheumatoid arthritis with a new mechanism of action, leflunomide, was recently put on the market by the company Aventis under the tradename ARAVA [EP 780128, WO 97/34600]. Leflunomide has immunomodulatory as well as anti-inflammatory properties [EP 217206, DE 2524929]. The mechanism of action is based upon the inhibition of dihydroorotate dehydrogenase (DHODH), an enzyme of the pyrimidine biosynthesis.

In the body, DHODH catalyzes the synthesis of pyrimidines, which are necessary for cell growth. An inhibition of DHODH inhibits the growth of (pathologically) fast proliferating cells, whereas cells which grow at normal speed may obtain their required pyrimidine bases from the normal metabolic cycle. The most important types of cells for the immuno response, the lymphocytes, use exclusively the synthesis of pyrimidines for their growth and react particularly sensitively to DHODH inhibition. Substances that inhibit the growth of lymphocytes are important medicaments for the treatment of auto-immuno diseases.

The DHODH inhibiting leflunomide (ARAVA) is the first medicament of this class of compounds (leflunomides) for the treatment of rheumatoid arthritis. WO 99/45926 is a further reference that discloses compounds which act as inhibitors of DHODH. WO 01/85685 discloses heterocylic derivatives such as thiadiazolidindiones for the treatment of a disease in which GSK-3 is involved, including Alzheimer's disease or the non-dependent insulin diabetes mellitus, or hyperprofilerative disease such as cancer, arterosclerosis or restenosis.

In WO 99/65867 a number of five or six membered cyclic hydroxamic acids are described.
In WO 03/006443 three or five membered cyclic acids are described.
In JP 02023337 , JP 02022650 cyclic acide derivatives are used as photographic material and in EP 74950 dicarboxylic acide amides are used as activator precursors in heat-developable diazo coupling systems.

It is an object of the present invention to provide alternative effective agents which can be used for the treatment of diseases which require the inhibition of DHODH.

Accordingly, a novel class of compounds with an inhibitory effect on DHODH, in particular human DHODH, was found.

The present invention is therefore directed to compounds of the general formula (I) 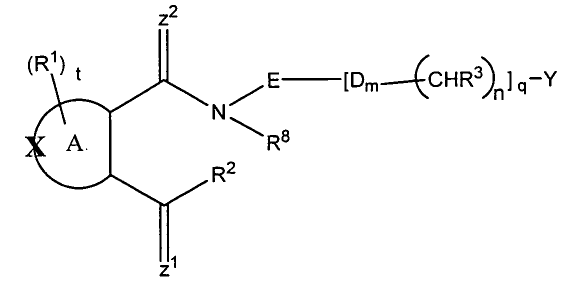 or salts or isomeres thereof, wherein
A is a 4-8 membered non-aromatic ring system, wherein one or more of the carbon atoms in the ring can be replaced by a group X, wherein X is selected from the group consisting of S, O, N, NR⁴, SO, CO or SO₂;
D is O, S, SO₂, NR⁴ or CH₂;
Z¹ and Z² are independent from each other O, S, or NR⁵ ;
R¹ independently represents H, halogen, haloalkyl, haloalkyloxy -CO₂R'', -SO₃H, -OH, -CONR*R'', -CR''O, -SO₂-NR*R'', -NO₂, -SO₂-R'', -SO-R*, -CN, alkoxy, alkylthio, aryl, -NR''-CO₂-R', -NR''-CO-R*, -NR''-SO₂-R', -O-CO-R*, -NR*R'', -NR*OR''-O-CO₂-R*, -O-CO-NR*R''; cycloalkyl, alkylamino, hydroxyalkylamino, -SH, heteroaryl, or alkyl;
R* independently represents H, alkyl, cycloalkyl, aminoalkyl, alkoxy, -OH, -SH, alkylthio, hydroxyalkyl, haloalkyl, haloalkyloxy, aryl or heteroaryl;
R' independently represents H, -CO₂R'', -CONHR", -CR''O, -SO₂NR'', -NR"-CO-haloalkyl, -NO₂, -NR''-SO₂-haloalkyl, -NR''-SO₂-alkyl, -SO₂-alkyl, -NR"-CO-alkyl, -CN, alkyl, cycloalkyl, aminoalkyl, alkylamino, alkoxy, -OH, -SH, -NR''R*, -NR''OR*, alkylthio, hydroxyalkyl, hydroxyalkylamino, halogen, haloalkyl, haloalkyloxy, aryl, arylalkyl or heteroaryl;
R" independently represents hydrogen, haloalkyl, hydroxyalkyl, alkyl, cycloalkyl, aryl, heteroaryl or aminoalkyl;
R² is H, OR⁶, NHR⁷, or R² togehter with the nitrogen atom to which R⁸ is attached forms a 5 or 6 membered heterocyclic ring with the proviso that R² is -[CH₂]₀₋₃ and R⁸ is absent;
R³ is H, alkyl, cycloalkyl, aryl, arylalkyl, alkoxy, O-aryl; O-cycloalkyl, halogen, aminoalkyl, alkylamino, hydroxylamino, hydroxylalkyl, haloalkyl, haloalkyloxy, heteroaryl, alkylthio, S-aryl, or S-cycloalkyl;
R⁴ is H, alkyl, cycloalkyl, aryl, or heteroaryl;
R⁵ is H, OH, alkoxy, O-aryl, alkyl, or aryl;
R⁶ is H, alkyl, cycloalkyl, aryl, heteroaryl, arylalkyl, alkylaryl, alkoxyalkyl, acylmethyl, (acyloxy)alkyl, non-symmetrical (acyloxy)alkyldiester, or dialkylphosphate;
R⁷ is H, alkyl, aryl, alkoxy, O-aryl, cycloalkyl, or O-cycloalkyl;
R⁸ is hydrogen or alkyl;
E is an alkyl or cycloalkyl group or a monocyclic or polycyclic substituted or unsubstituted ring system which may contain one or more groups X and which contains at least one aromatic ring;
Y is hydrogen, halogen, haloalkyl, haloalkyloxy, alkyl, cycloalkyl, a monocyclic or polycyclic substituted or unsubstituted ring system which may contain one or more groups X and which contains at least one aromatic ring or 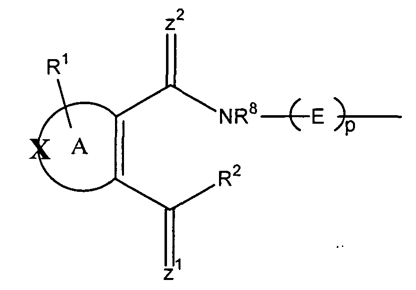
m is 0 or 1;
n is 0 or 1;
p is 0 or 1;
q is 0 or 1;
t is 1 to 3;
with the proviso that trans-2-[4-(Naphthalin-2-yl)thiazol-2-ylaminocarbonyl]cyclopentane carboxylic acid is excluded;
an alkyl group, if not stated otherwise, denotes a linear or branched C₁-C₆-alkyl, preferably a linear or branched chain of one to five carbon atoms, a linear or branched C₁-C₆-alkenyl or a linear or branched C₁-C₆-alkinyl group, which can optionally be substituted by one or more substituents R', preferably by halogen;
the C₁-C₆-alkyl, C₁-C₆-alkenyl and C₁-C₆-alkinyl residue may be selected from the group comprising -CH₃, -C₂H₅, -CH=CH₂, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R')₃, -CH₂-C(R')₃, -C₂H₄-C(R')₃, -C₂H₄-CH-CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C=CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇,-C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅;
R' being as defined above; R" being as defined above;
a cycloalkyl group denotes a non-aromatic ring system containing three to eight carbon atoms, preferably four to eight carbon atoms, wherein one or more of the carbon atoms in the ring can be substituted by a group X, X being as defined above; the C₃-C₈-cycloalkyl residue may be selected from the group comprising -cyclo-C₃H₅, -cyclo-C₄H₇, -cyclo-C₅H₉, -cyclo-C₆H₁₁, -cyclo-C₇H₁₃, -cyclo-C₈H₁₅;
an alkoxy group denotes an O-alkyl group, the alkyl group being as defined above; the alkoxy group is preferably a methoxy, ethoxy, isopropoxy, t-butoxy or pentoxy group;
an alkylthio group denotes an S-alkyl group, the alkyl group being as defined above.
an haloalkyl group denotes an alkyl group which is substituted by one to five halogen atoms, the alkyl group being as defined above; the haloalkyl group is preferably a -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇ or -C₂H₄-C(R¹⁰)₃, wherein R¹⁰, R^{10'}, R^{10"} represent F, Cl, Br or I, preferably F;
a hydroxyalkyl group denotes an HO-alkyl group, the alkyl group being as defined above;
an haloalkyloxy group denotes an alkoxy group which is substituted by one to five halogen atoms, the alkyl group being as defined above; the haloalkyloxy group is preferably a -OC(R¹⁰)₃, -OCR¹⁰(R^{10'})₂, -OCR¹⁰(R^{10'})R^{10"}, -OC₂(R¹⁰)₅, -OCH₂-C(R¹⁰)₃, -OCH₂-CR¹⁰(R^{10'})₂, -OCH₂-CR¹⁰(R^{10'})R^{10"}, -OC₃(R¹⁰)₇ or -OC₂H₄-C(R¹⁰)₃, wherein R¹⁰, R^{10'}, R^{10"} represent F, Cl, Br or I, preferably F;
a hydroxyalkylamino group denotes an (HO-alkyl)₂-N- group or HO-alkyl-NH- group, the alkyl group being as defined above;
an alkylamino group denotes an HN-alkyl or N-dialkyl group, the alkyl group being as defined above;
a halogen group is chlorine, bromine, fluorine or iodine, fluorine being preferred;
an aryl group preferably denotes an aromatic group having five to fifteen carbon atoms, which can optionally be substituted by one or more substituents R', where R' is as defined above; the aryl group is preferably a phenyl group, -CH₂Ph, -C₂H₄Ph, -CH=CH-Ph, -C≡C-Ph, -o-C₆H₄- R', -m-C₆H₄- R', -p-C₆H₄- R', -o-CH₂-C₆H₄- R', -m-CH₂-C₆H₄-R', -p-CH₂-C₆H₄- R', or 1-naphthyl, 2-naphthyl, 1-anthracyl, 2-anthracyl optionally substituted by one or more R', where R' is as defined above;
a heteroaryl group represents a 5- or 6-membered heterocyclic group which contains at least one heteroatom like O, N, or S. This heterocyclic group can be fused to another ring. For example, this group can be selected from a thiazole-2-yl, thiazole-4-yl, thiazole-5-yl, isothiazole-3-yl, isothiazole-4-yl, isothiazole-5-yl, 1,2,4-oxadiazole-3-yl, 1,2,4-oxadiazole-5-yl, 1,2,4-thiadiazole-3-yl, 1,2,4-thiadiazole-5-yl, 1,2,5-oxadiazole-3-yl, 1,2,5-oxadiazole-4-yl, 1,2,5-thiadiazole-3-yl, 1-imidazolyl, 2-imidazolyl, 1,2,5-thiadiazol-4-yl, 4-imidazolyl, 1-pyrrolyl, 2-pyrrolyl, 3-pyrrolyl, 1-pyrrolididnyl, 2-pyrrolidinyl, 3-pyrrolidinyl, 2-furanyl, 3-furanyl, 2-thienyl, 3-thienyl, 2-pyridyl, 3-pyridyl, 4-pyridyl, 2-pyrimidyl, 4-pyrimidyl, 5-pyrimidyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, isoindolyl, indolinyl, benzofuranyl, benzo[b]thiophenyl, benzo[b]thienyl, benzooxazolyl, benzoisoxazolyl, benzotriazolyl, benzimidazolyl, benzothiazolyl, quinazolinyl, quinoxazolinyl, oxazole-2-yl, oxazole-4-yl, oxazole-5-yl, oxadizole-2-yl, oxadiazole-5-yl, isoxazole-3-yl, isoxazole-4-yl, isoxazole-5-yl, acridinyl, tetrazol-5-yl, triazol-2-yl, carbazolyl, piperidin-1-yl, piperidin-2-yl, piperidin-3-yl, piperidin-4-yl, quinolinyl, tetrahydroquinolinyl, isoquinolinyl, tetrahydroisoquinolinyl, pyrido[3,4-d]pyrimidinyl, 5-phenyl-furan-2-yl, 5-phenyl-furan-3-yl, 4-phenyl-furan-2-yl, 4-phenyl-furan-3-yl group. This heterocyclic group can optionally be substituted by one or more substituents R', where R' is as defined above.

The present invention is also directed to compounds of the general formula (II) 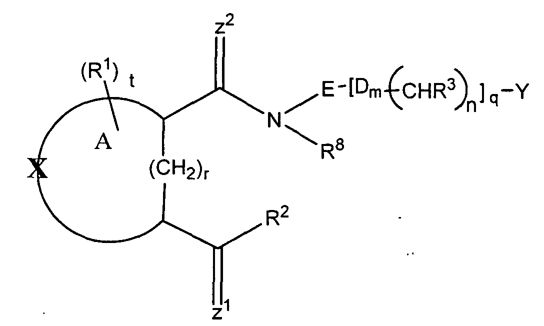 or salts or isomeres thereof, wherein
A is a 3-8 membered non-aromatic ring system, wherein the ring system comprises at least one double bond and wherein one or more of the carbon atoms in the ring can be replaced by a group X, wherein X is selected from the group consisting of S, O, N, NR⁴, SO, CO or SO₂, wherein, when r = 0, there is no double bond between the carbon atoms carrying the substituents -CZ'- and -CZ²-;
D is O, S, SO₂, NR⁴ or CH₂;
Z¹ and Z² are independent from each other O, S, or NR⁵ ;
R¹ independently represents H, halogen, haloalkyl, haloalkyloxy -CO₂R'', -SO₃H, -OH, -CONR*R'', -CR''O, -SO₂-NR*R'', -NO₂, -SO₂-R'', -SO-R*, -CN, alkoxy, alkylthio, aryl, -NR''-CO₂-R', -NR''-CO-R*, -NR''-SO₂-R', -O-CO-R*, -NR*R'', -NR*OR''-O-CO₂-R*, -O-CO-NR*R''; cycloalkyl, alkylamino, hydroxyalkylamino, -SH, heteroaryl, or alkyl;
R* independently represents H, alkyl, cycloalkyl, aminoalkyl, alkoxy, -OH, -SH, alkylthio, hydroxyalkyl, haloalkyl, haloalkyloxy, aryl or heteroaryl;
R' independently represents H, -CO₂R'', -CONHR", -CR''O, -SO₂NR'', -NR"-CO-haloalkyl, -NO₂, -NR''-SO₂-haloalkyl, -NR''-SO₂-alkyl, -SO₂-alkyl, -NR"-CO-alkyl, -CN, alkyl, cycloalkyl, aminoalkyl, alkylamino, alkoxy, -OH, -SH, -NR''R*, -NR''OR*, alkylthio, hydroxyalkyl, hydroxyalkylamino, halogen, haloalkyl, haloalkyloxy, aryl, arylalkyl or heteroaryl;
R" independently represents hydrogen, haloalkyl, hydroxyalkyl, alkyl, cycloalkyl, aryl, heteroaryl or aminoalkyl;
R² is H, OR⁶, NHR⁷, NHOR⁶ or R² togehter with the nitrogen atom to which R⁸ is attached forms a 5 or 6 membered heterocyclic ring with the proviso that R² is -[CH₂]₀₋₃ and R⁸ is absent;
R³ is H, alkyl, cycloalkyl, aryl, arylalkyl, alkoxy, O-aryl; O-cycloalkyl, halogen, aminoalkyl, alkylamino, hydroxylamino, hydroxylalkyl, haloalkyl, haloalkyloxy, heteroaryl, alkylthio, S-aryl, or S-cycloalkyl;
R⁴ is H, alkyl, cycloalkyl, aryl, or heteroaryl;
R⁵ is H, OH, alkoxy, O-aryl, alkyl, or aryl;
R⁶ is H, alkyl, cycloalkyl, aryl, heteroaryl, arylalkyl, alkylaryl, alkoxyalkyl, acylmethyl, (acyloxy)alkyl, non-symmetrical (acyloxy)alkyldiester, or dialkylphosphate;
R⁷ is H, alkyl, aryl, alkoxy, O-aryl, cycloalkyl, or O-cycloalkyl;
R⁸ is hydrogen or alkyl;
E is an alkyl or cycloalkyl group or a monocyclic or polycyclic substituted or unsubstituted ring system which may contain one or more groups X and which contains at least one aromatic ring;
Y is hydrogen, halogen, haloalkyl, haloalkyloxy, alkyl, cycloalkyl, a monocyclic or polycyclic substituted or unsubstituted ring system which may contain one or more groups X and which contains at least one aromatic ring or 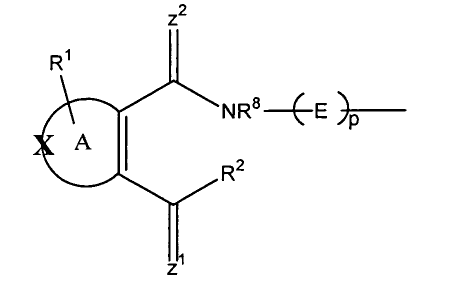
m is 0 or 1;
n is 0 or 1;
p is 0 or 1;
r is 0 or 1; and
q is 0 or1;
t is 1 to 3;
an alkyl group, if not stated otherwise, denotes a linear or branched C₁-C₆-alkyl, preferably a linear or branched chain of one to five carbon atoms, a linear or branched C₁-C₆-alkenyl or a linear or branched C₁-C₆-alkinyl group, which can optionally be substituted by one or more substituents R', preferably by halogen;
the C₁-C₆-alkyl, C₁-C₆-alkenyl and C₁-C₆-alkinyl residue may be selected from the group comprising -CH₃, -C₂H₅, -CH=CH₂, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R')₃, -C₂(R')₅, -CH₂-C(R')₃, -C₃(R')₇, -C₂H₄-C(R')₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C=CH, -C=C-C=CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C=CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇,-C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅;
R' being as defined above; R" being as defined above;
a cycloalkyl group denotes a non-aromatic ring system containing three to eight carbon atoms, preferably four to eight carbon atoms, wherein one or more of the carbon atoms in the ring can be substituted by a group X, X being as defined above; the C₃-C₈-cycloalkyl residue may be selected from the group comprising -cyclo-C₃H₅, -cyclo-C₄H₇, -cyclo-C₅H₉, -cyclo-C₆H₁₁, -cyclo-C₇H₁₃, -cyclo-C₈H₁₅;
an alkoxy group denotes an O-alkyl group, the alkyl group being as defined above; the alkoxy group is preferably a methoxy, ethoxy, isopropoxy, *t*-butoxy or pentoxy group;
an alkylthio group denotes an S-alkyl group, the alkyl group being as defined above.
an haloalkyl group denotes an alkyl group which is substituted by one to five halogen atoms, the alkyl group being as defined above; the haloalkyl group is preferably a -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇ or -C₂H₄-C(R¹⁰)₃, wherein R¹⁰, R^{10'}, R^{10"} represent F, Cl, Br or I, preferably F;
a hydroxyalkyl group denotes an HO-alkyl group, the alkyl group being as defined above; an haloalkyloxy group denotes an alkoxy group which is substituted by one to five halogen atoms, the alkyl group being as defined above; the haloalkyloxy group is preferably a -OC(R¹⁰)₃, -OCR¹⁰(R^{10'})₂, -OCR¹⁰(R^{10'})R^{10"}, -OC₂(R¹⁰)₅, -OCH₂-C(R¹⁰)₃, -OCH₂-CR¹⁰(R^{10'})₂, -OCH₂-CR¹⁰(R^{10'})R^{10"}, -OC₃(R¹⁰)₇ or -OC₂H₄-C(R¹⁰)₃, wherein R¹⁰, R^{10'}, R^{10"} represent F, Cl, Br or I, preferably F;
a hydroxyalkylamino group denotes an (HO-alkyl)₂-N- group or HO-alkyl-NH- group, the alkyl group being as defined above;
an alkylamino group denotes an HN-alkyl or N-dialkyl group, the alkyl group being as defined above;
a halogen group is chlorine, bromine, fluorine or iodine, fluorine being preferred;
an aryl group preferably denotes an aromatic group having five to fifteen carbon atoms, which can optionally be substituted by one or more substituents R', where R' is as defined above; the aryl group is preferably a phenyl group, -CH₂Ph, -C₂H₄Ph, -CH=CH-Ph, -C=C-Ph, -o-C₆H₄- R', -m-C₆H₄- R', -p-C₆H₄- R', -o-CH₂-C₆H₄- R', -m-CH₂-C₆H₄-R', -p-CH₂-C₆H₄- R', or 1-naphthyl, 2-naphthyl, 1-anthracyl, 2-anthracyl optionally substituted by one or more R', where R' is as defined above;
a heteroaryl group represents a 5- or 6-membered heterocyclic group which contains at least one heteroatom like O, N, or S. This heterocyclic group can be fused to another ring. For example, this group can be selected from a thiazole-2-yl, thiazole-4-yl, thiazole-5-yl, isothiazole-3-yl, isothiazole-4-yl, isothiazole-5-yl, 1,2,4-oxadiazole-3-yl, 1,2,4-oxadiazole-5-yl, 1,2,4-thiadiazole-3-yl, 1,2,4-thiadiazole-5-yl, 1,2,5-oxadiazole-3-yl, 1,2,5-oxadiazole-4-yl, 1,2,5-thiadiazole-3-yl, 1-imidazolyl, 2-imidazolyl, 1,2,5-thiadiazol-4-yl, 4-imidazolyl, 1-pyrrolyl, 2-pyrrolyl, 3-pyrrolyl, 1-pyrrolididnyl, 2-pyrrolidinyl, 3-pyrrolidinyl, 2-furanyl, 3-furanyl, 2-thienyl, 3-thienyl, 2-pyridyl, 3-pyridyl, 4-pyridyl, 2-pyrimidyl, 4-pyrimidyl, 5-pyrimidyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, isoindolyl, indolinyl, benzofuranyl, benzo[b]thiophenyl, benzo[b]thienyl, benzooxazolyl, benzoisoxazolyl, benzotriazolyl, benzimidazolyl, benzothiazolyl, quinazolinyl, quinoxazolinyl, oxazole-2-yl, oxazole-4-yl, oxazole-5-yl, oxadizole-2-yl, oxadiazole-5-yl, isoxazole-3-yl, isoxazole-4-yl, isoxazole-5-yl, acridinyl, tetrazol-5-yl, triazol-2-yl, carbazolyl, piperidin-1-yl, piperidin-2-yl, piperidin-3-yl, piperidin-4-yl, quinolinyl, tetrahydroquinolinyl, isoquinolinyl, tetrahydroisoquinolinyl, pyrido[3,4-d]pyrimidinyl, 5-phenyl-furan-2-yl, 5-phenyl-furan-3-yl, 4-phenyl-furan-2-yl, 4-phenyl-furan-3-yl group. This heterocyclic group can optionally be substituted by one or more substituents R', where R' is as defined above.

The meaning of E in formula (I) or in formula (II) includes alkyl groups optionally substituted by one or more substituents R', wherein alkyl is defined as above or as a cycloalkyl group optionally substituted by one or more substituents R' such as cyclopropyl, cyclobutyl, cyclopentyl, cyclohexyl, cycloheptyl, cyclooctyl or carbocyclic aromatic groups such as phenyl, 1-naphthyl, 2-naphthyl, anthracenyl, in particular 1-anthracenyl and 2-anthracenyl, and heterocyclic aromatic groups such as N-imidazolyl, 2-imidazolyl, 2-thienyl, 3-thienyl, 2-furanyl, 3-furanyl, 2-pyridyl, 3-pyridyl, 4-pyridyl, 2-pyrimidyl, 4-pyrimidyl, 2-pyranyl, 3-pyranyl, 4-pyranyl, 3-pyrazolyl, 4-pyrazolyl, 5-pyrazolyl, 2-pyrazinyl, 2-thiazolyl, 4-thiazolyl, 5-thiazolyl, 2-oxazolyl, 4-oxazolyl and 5-oxazolyl. E includes also fused polycyclic aromatic ring systems such as 9H-thioxanthene-10,10-dioxide in which a carbocyclic aromatic ring or heteroaryl ring is fused to one or more other heteroaryl ring.

The invention also provides a pharmaceutical composition comprising a compound of formula (I) or of formula (II) in free form or in the form of pharmaceutically acceptable salts and physiologically functional derivatives, together with a pharmaceutically acceptable diluent or carrier therefore.

The term "physiologically functional derivative" as used herein refers to compounds which are not pharmaceutically active themselves but which are transformed into their pharmaceutical active form in vivo, i.e. in the subject to which the compound is administered. Examples of physiologically functional derivatives are prodrugs such as those described below in the present application.

In another aspect, the present invention also provides a method for the treatment or prophylaxis of a condition where there is an advantage in inhibiting dihydroorotate dehydrogenase (DHODH) which comprises the administration of an effective amount of a compound of formula (I) or of formula (II) and physiologically acceptable salts or physiologically functional derivatives thereof.

The invention is also directed to the use of compounds of the formula (I) or of formula (II) and of their pharmacologically tolerable salts or physiologically functional derivatives for the production of a medicament for the prevention and treatment of diseases, where inhibition of the pyrimidine biosynthesis is of benefit.

In addition, the present invention provides methods for preparing the compounds of the invention such as compounds of formula (I) or of formula (II).

The compounds of formula (I) may be obtained via various methods. Hydration of formula (III) with a catalysator like platin oxide resulted in formula (I).

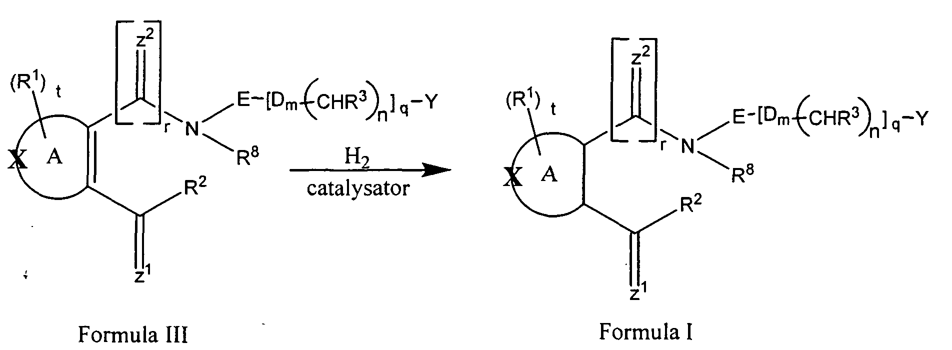

Another method for the synthesis of compounds of formula (I) [r=1] is reacting the anhydride of formula (IV) with the amine of formula (V).

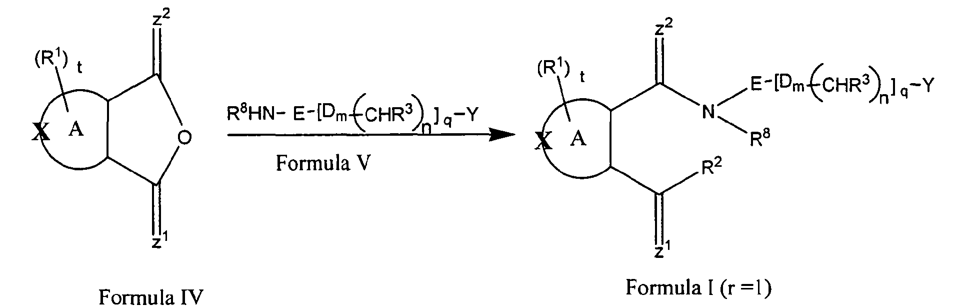

For the synthesis of compounds of formula (II) (wherein ring A is a five membered ring system and r = 0) compounds of formula (VI) were reacted with base like butyllitium.

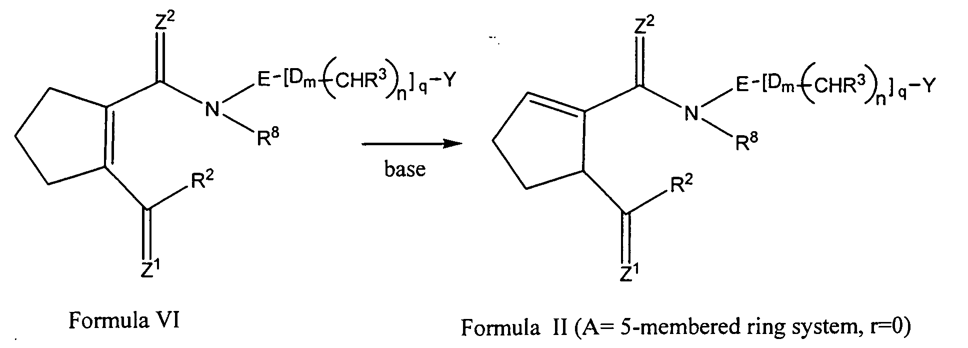

Alternative synthesis of compounds of formula (II) (wherein r=0) can be preformed, starting with the compound of formula (VII) which synthesis was described by H. C. Stevens; G.M. Trenta, *J. Chem. Com*. **1970,** 1407-1408 or D. Nöteberg; J. Brånalt; *J*. *Med. Chem*. **2000,** 43, 1705-1713 or F. Thorstensson; I. Kvarnström; *J*. *Med. Chem*. **2003,** 46, 1165-117.

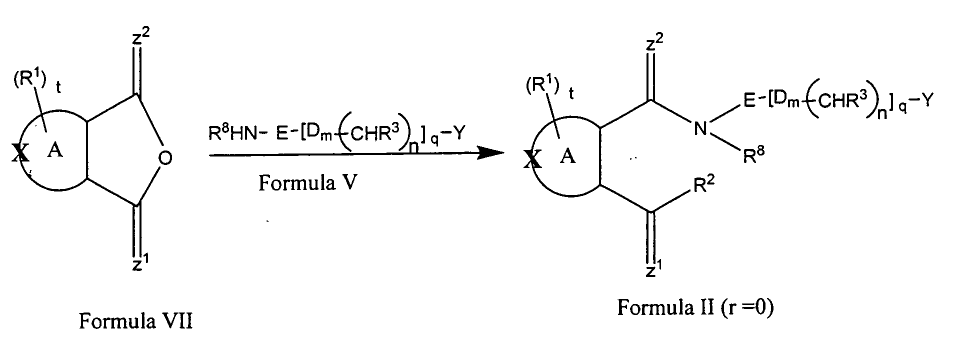

In the compounds of formula (I), the non-aromatic ring system A contains 4, 5, 6, 7 or 8, preferably 5 carbon atoms. One or more of the carbon atoms in the ring system A can be replaced by a group X, wherein X is selected from the group consisting of S, O, N, NR⁴, SO, CO or SO₂. In one preferred embodiment, one carbon atom is replaced by a group X=S, X=CO, or X=O. In a more preferred embodiment, none of the carbon atoms is replaced by a group X. In another preferred embodiment, in the compounds of formula (I), A is 1-cyclopentan-1,2-diyl, 1-cyclohexan-1,2-diyl, 1-cycloheptan-1,2-diyl.

In the compounds of formula (II), the non-aromatic ring system A is a 3-8 membered non-aromatic ring system, wherein the ring system comprises at least one double bond and wherein one or more of the carbon atoms in the ring can be replaced by a group X, wherein X is selected from the group consisting of S, O, N, NR⁴, SO, CO or SO₂; wherein, when r = 0, there is no double bond between the carbon atoms carrying the substituents -CZ'- and -CZ²-;

In another preferred embodiment, in the compounds of formula (II), A is

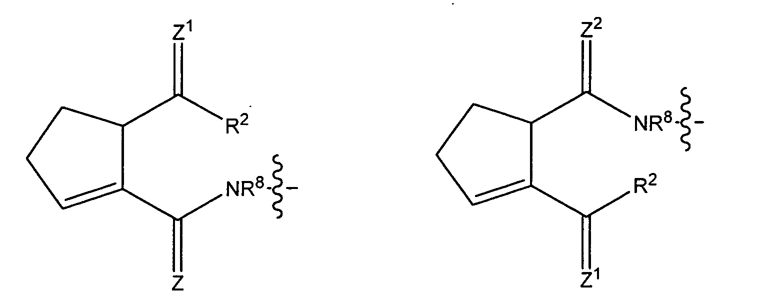

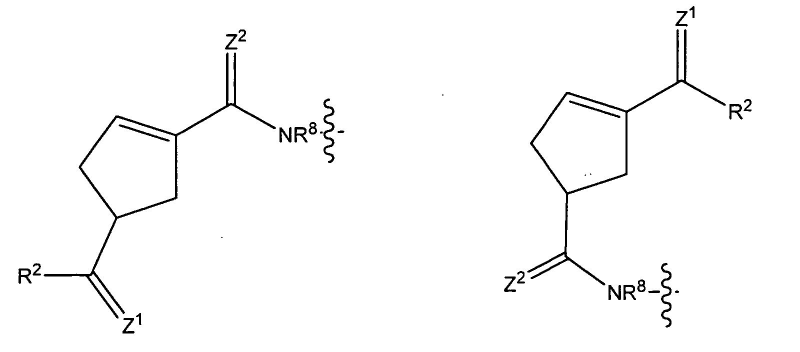

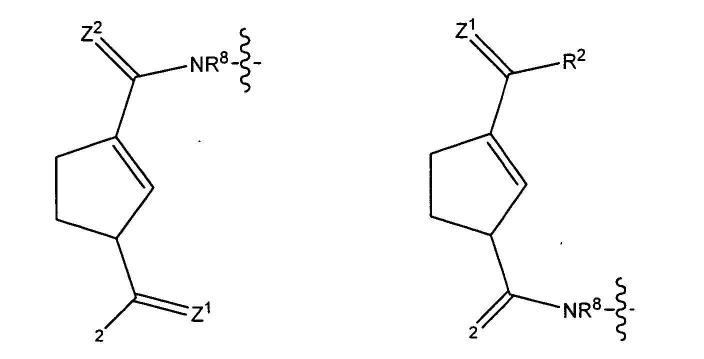

In the compounds of formula (I) or of formula (II), R' is preferably H, OH or CO₂H or SO₃H.
In the compounds of formula (I) or of formula (II), R² is H, OR⁶, preferably OH or OR⁶.

In preferred embodiments, in the compounds of formula (I) or of formula (II), R⁶ is benzoyloxymethyl, isobutyryloxymethyl, 4-amino-butyryloxymethyl, butyryloxymethyl, 1-(butyryloxy)ethyl, 1-(butyryloxy)-2,2-dimethylpropyl, 1-diethylphosphonooxyethyl, 2-(2-methoxyethoxy)-acetyloxymethyl, p-aminobenzoylmethyl, nicotinyloxymethyl, pivalyloxymethyl, glutaryloxymethyl, [2-(2-methoxyethoxy)ethoxy]-acetyloxymethyl, 2-(morpholine-4-yl)-ethyl, 1-diethyl-phosphonooxymethyl.

In the compounds of formula (I) or of formula (II), R³ is is H, alkyl, cycloalkyl, aryl, alkoxy, O-aryl; O-cycloalkyl, halogen, aminoalkyl, akylamino, hydroxylamino, haloalkyl, hydroxylalkyl, haloalkyloxy, heteroaryl, alkylthio, S-aryl; S-cycloalkyl, arylalkyl, preferably H.

In the compounds of formula (I) or of formula (II), R⁴ is H, alkyl, cycloalkyl, aryl or heteroaryl, preferably H.

In formula (I) or of formula (II), R⁸ is H or alkyl, preferably H or methyl.

In formula (I) or of formula (II), Z¹ and Z² are independent from each other O, S, or NR⁵, preferably both are O.

In formula (I) or of formula (II), Y is hydrogen, halogen, alkyl, substituted or unsubstituted cycloalkyl, substituted or unsubstituted E, substituted or unsubstituted O-E, substituted or unsubstituted O-alkylaryl, substituted or unsubstituted O-arylalkyl; in case of said substitution, substitution of one or more hydrogen atoms of the alkyl-, cycloalkyl-, or aryl-groups by halogens are preferred. Y can also be 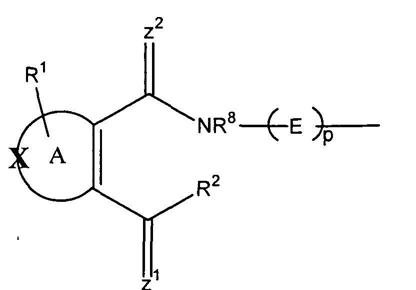 wherein A, X, R¹, R², R⁸, Z¹, Z² and p have the meaning as defined above. Preferably Y is E and more preferably Y is an optionally substituted phenyl.

In formula (I) or of formula (II), E is an alkyl or cycloalkyl group which is optionally substituted by one or more substituents R', or E is a monocyclic or polycyclic substituted or unsubstituted ring system which contains at least one aromatic ring and which may also contain one or more groups X selected from S, O, N, NR⁴, SO or SO₂. In preferred embodiments, E is a monocyclic aromatic ring or an aromatic bicyclic or tricyclic ring system, or cycloalkyl. In case of substitutions of carbon atoms in the ring system, preferably one, two or three carbon atoms are replaced by a group X as defined above.

In formula (I) or of formula (II), E is preferably an optionally by one or more substituents R' substituted phenyl, 1-naphtyl, 2-naphthyl, 1-anthracyl and 2-anthracyl.

In a preferred embodiment of the present invention in compounds of formula (I) or of formula (II), E is an optionally by one or more substituents R' substituted phenyl , or an optionally by one or more substituents R' substituted cycloalkyl.

In formula (I) or of formula (II), preferred substituents R' are nitro, halogen, alkoxy, haloalkyl, haloalkyloxy, heteroaryl, alkyl or aryl, more preferably R' is Br, F, Cl, CF₃, OCF₃, methoxy or ethoxy.

In formula (I) or of formula (II), preferred heteroaryl groups are imidazoyl, thienyl, furanyl, pyridyl, pyrimidyl, pyranyl, pyrazolyl, pyrazinyl, thiazolyl, 1H-tetrazol-2-yl, 1H-tetrazol-3-yl, or oxazolyl.

In formula (I) or of formula (II), t is preferably 1 or 2.
In formula (II), r is preferably 0.

In the compounds of formula (I) or of formula (II), D is O, S, SO₂, NR⁴, or CH₂. D is preferably S or more preferably O, when m = 1.

In other preferred embodiment, in the compounds of formula (I) or of formula (II), m and q are zero and Y is hydrogen, halogen, haloalkyl, haloalkyloxy, alkyl, cycloalkyl or E, preferably F, CF₃, OCF₃, an optionally by one or more substituents R' substituted phenyl or more preferably an optionally by one or more F, Cl, methoxy, CF₃, or OCF₃ substituted phenyl.

In particular preferred embodiment of the invention, in compounds of formula (I) the substituents -CZ1- and -CZ2- of ring A have trans conformation.

In particular preferred are compounds of formula (I) or of formula (II) wherein Y and -CNR⁸ are in para-position on E.

In particular preferred embodiment of the invention, in compounds of formula (I), q=0, t=1, and A is a carbocyclic non-aromatic ring system, Y is H or F, or E is phenyl which is either unsubstituted or substituted with Cl, F and/or CF₃ or OCF₃.

In another particularly preferred embodiment of the invention, in compounds of formula (I), q=0, t=1, and A is a carbocyclic non-aromatic ring system, and E and Y are substituted or unsubstituted phenylene and phenyl, respectively.

In further particularly preferred embodiment, in compounds of formula (I), D=O (thus m=1), R³ is H (thus n=1), q=1, t=1, and E is phenylene which is either unsubstituted or substituted with Cl, F and/or CF₃ or OCF₃, and Y is phenyl which is also either unsubstituted or substituted with Cl, F and/or CF₃ or OCF₃, and A is a carbocyclic non-aromatic ring system.

In further particularly preferred embodiment, in compounds of formula (I) D=O (thus m=1), n=0, q=1, t = 1, and E is phenylene which is either unsubstituted or substituted with Cl, F and/or CF₃ or OCF₃, and Y is phenyl which is also either unsubstituted or substituted with Cl, F and/or CF₃ or OCF₃, and A is a carbocyclic non-aromatic ring system.

In further particularly preferred embodiment, in compounds of formula (I), D=S (thus m=1), n=0, q=1, t=1, and E is phenylene which is either unsubstituted or substituted with Cl, F and/or CF₃ or OCF₃, and Y is phenyl which is also either unsubstituted or substituted with Cl, F and/or CF₃ or OCF₃, and A is a carbocyclic non-aromatic ring system.

In particular preferred embodiment of the invention, in compounds of formula (I), q=0, t=1, and A is a non-aromatic ring system, wherein one carbon atom is replaced by O, or Y is H or F, and E is phenyl which is either unsubstituted or substituted with Cl, F and/or CF₃ or OCF₃.

In another particularly preferred embodiment of the invention, in compounds of formula (I), q=0, t=1, and A is a non-aromatic ring system, wherein one carbon atom is replaced by O, or E and Y are substituted or unsubstituted phenylene and phenyl, respectively.

In further particularly preferred embodiment, in compounds of formula (I), D=O (thus m=1), R³ is H (thus n=1), q=1 or 2, t=1, and E is phenylene which is either unsubstituted or substituted with Cl, F and/or CF₃ or OCF₃, and Y is phenyl which is also either unsubstituted or substituted with Cl, F and/or CF₃ or OCF₃, and A is a non-aromatic ring system, wherein one carbon atom is replaced by O.

In further particularly preferred embodiment, in compounds of formula (I), D=O (thus m=1), n=0, q=1, t=1, and E is phenylene which is either unsubstituted or substituted with Cl, F and/or CF₃ or OCF₃, and Y is phenyl which is also either unsubstituted or substituted with Cl, F and/or CF₃ or OCF₃, and A is a non-aromatic ring system, wherein one carbon atom is replaced by O.

In further particularly preferred embodiment, in compounds of formula (I), D=S (thus m=1), n=0, q=1, t=1, and E is phenylene which is either unsubstituted or substituted with Cl, F and/or CF₃ or OCF₃, and Y is phenyl which is also either unsubstituted or substituted with Cl, F and/or CF₃ or OCF₃, and A is a non-aromatic ring system, wherein one carbon atom is replaced by O.

In particular preferred embodiment of the invention, in compounds of formula (I), q=0, t=1, and A is a non-aromatic ring system, wherein one carbon atom is replaced by S, or Y is H or F, and E is phenyl which is either unsubstituted or substituted with Cl, F and/or CF₃ or OCF₃.

In another particularly preferred embodiment of the invention, in compounds of formula (I), q=0, t=1, and A is a non-aromatic ring system, wherein one carbon atom is replaced by S, or E and Y are substituted or unsubstituted phenylene and phenyl, respectively.

In further particularly preferred embodiment, in compounds of formula (I), D=O (thus m=1), R³ is H (thus n=1), q=1 or 2, t=1, and E is phenylene which is either unsubstituted or substituted with Cl, F and/or CF₃ or OCF₃, and Y is phenyl which is also either unsubstituted or substituted with Cl, F and/or CF₃ or OCF₃, and A is a non-aromatic ring system, wherein one carbon atom is replaced by S.

In further particularly preferred embodiment, in compounds of formula (I), D=O (thus m=1), n=0, q=1 or 2, t=1, and E is phenylene which is either unsubstituted or substituted with Cl, F and/or CF₃ or OCF₃, and Y is phenyl which is also either unsubstituted or substituted with Cl, F and/or CF₃ or OCF₃, and A is a non-aromatic ring system, wherein one carbon atom is replaced by S.

In further particularly preferred embodiment, in compounds of formula (I), D=S (thus m=1), n=0, q=1, t=1, and E is phenylene which is either unsubstituted or substituted with Cl, F and/or CF₃ or OCF₃, and Y is phenyl which is also either unsubstituted or substituted with Cl, F and/or CF₃ or OCF₃, and A is a non-aromatic ring system, wherein one carbon atom is replaced by S.

In formula (I), q is 0 or 1, preferably q is 0. If q is 1 and n is 0 or 1, D is preferably O (thus m=1).

In particular preferred embodiment of the invention, in compounds of formula (I), t=1, Z¹=O, Z²=O, X= CO, E is phenylene which is either unsubstituted or substituted with Cl, F and/or CF₃ or OCF₃, and Y is phenyl which is also either unsubstituted or substituted with Cl, F and/or CF₃ or OCF₃, and A is a five membered aromatic ring system. and R² togehter with the nitrogen atom which is attached to R⁸ form a 6 membered heterocyclic ring with the proviso that R² is -[CH₂]ₛ and R⁸ is absent; and A is a five membered carbocylic ring system.

In particular preferred embodiments of the invention, in compounds of formula (I), t=1, Z¹=O, Z²=O, X= CO, and E is phenylene which is either unsubstituted or substituted with Cl, F and/or CF₃ or OCF₃, and Y is H or F, and R² togehter with the nitrogen atom which is attached to R⁸ form a 6 membered heterocyclic ring with the proviso that R² is -[CH₂]ₛ and R⁸ is absent; and A is a five membered carbocylic ring system.

The compounds of the formula (I) or of formula (II), to be used according to the invention can form salts with inorganic or organic acids or bases. Examples of such salts are, for example, alkali metal salts, in particular sodium and potassium salts, or ammonium salts.

The compounds of the present invention can be used for a variety of human and animal diseases, preferably human diseases, where inhibition of the pyrimidine metabolism is beneficial. Such diseases are:
- fibrosis, uveitis, rhinitis, asthma or athropathy, in particular, arthrosis
- all forms of rheumatism
- acute immunological events and disorders such as sepsis, septic shock, endotoxic shock, Gram-negative sepsis, toxic shock sydrome, acute respiratory distress syndrome, stroke, reperfusion injury, CNS injury, serious forms of allergy, graft versus host and host versus graft reactions, alzheimer's or pyresis, restenosis, chronic pulmonary inflammatory disease, silicosis, pulmonary sarcosis, bone resorption disease. These immunological events also include a desired modulation and suppression of the immune system;
- all types of autoimmune diseases, in particular rheumatoid arthritis, rheumatoid spondylitis, osteoarthritis, gouty arthritis, multiple sclerosis, insulin dependent diabetes mellitus and non-insulin dependent diabetes, and lupus erythematoidis, ulcerative colitis, Morbus Crohn, inflammatory bowel disease, as well as other chronic inflammations, chronic diarrhea;
- dermatological disorders such as psoriasis
- progressive retinal atrophy
- all kinds of infections including opportunistic infections.

The compounds according to the invention and medicaments prepared therewith are generally useful for the treatment of cell proliferation disorders, for the treatment or prophylaxis, immunological diseases and conditions (as for instance inflammatory diseases, neuroimmunological diseases, autoimmune diseases or other).

The compounds of the present invention are also useful for the development of immunomodulatory and anti-inflammatory medicaments or, more generally, for the treatment of diseases where the inhibition of the pyrimidine biosynthesis is beneficial.

The compounds of the present invention are also useful for the treatment of diseases which are caused by malignant cell proliferation, such as all forms of hematological and solid cancer. Therefore the compounds according to the invention and medicaments prepared therewith are generally useful for regulating cell activation, cell proliferation, cell survival, cell differentiation, cell cycle, cell maturation and cell death or to induce systemic changes in metabolism such as changes in sugar, lipid or protein metabolism. They can also be used to support cell generation poiesis, including blood cell growth and generation (prohematopoietic effect) after depletion or destruction of cells, as caused by, for example, toxic agents, radiation, immunotherapy, growth defects, malnutrition, malabsorption, immune dysregulation, anemia and the like or to provide a therapeutic control of tissue generation and degradation, and therapeutic modification of cell and tissue maintenance and blood cell homeostasis.

These diseases and conditions include but are not limited to cancer as hematological (e.g. leukemia, lymphoma, myeloma) or solid tumors (for example breast, prostate, liver, bladder, lung, esophageal, stomach, colorectal, genitourinary, gastrointestinal, skin, pancreatic, brain, uterine, colon, head and neck, and ovarian, melanoma, astrocytoma, small cell lung cancer, glioma, basal and squameous cell carcinoma, sarcomas as Kaposi's sarcoma and osteosarcoma), treatment of disorders involving T-cells such as aplastic anemia and DiGeorge syndrome, Graves' disease.

Leflunomide, was previously found to inhibit HCMV replication in cell culture. Ocular herpes is the most common couse of infectious blindness in the developed world. There are about 50.000 cases per year in the US alone, of which 90% are recurences of initial infections. Recurrences are treated with antivirals and corticosteroids. Cytomegalovirus another herpes virus is a common couse of retinal damage and blindness in patients with aids. The compounds of the present invention can be used alone or in combination with other antiviral compounds such as Ganciclovir and Foscamet to treat such diseases.

The compounds of the present invention can further be used for diseases that are caused by protozoa infestations in humans and animals. Such veterinary and human pathogenic protozoas are preferably intracellular active parasites of the phylum Apicomplexa or Sarcomastigophora, especially Trypanosoma, Plasmodia, Leishmania, Babesia and Theileria, Cryptosporidia, Sacrocystida, Amoebia, Coccidia and Trichomonadia. These active substances or corresponding drugs are especially suitable for the treatment of Malaria tropica, caused by *Plasmodium falciparum*, Malaria tertiana, caused by *Plasmodium vivax* or *Plasmodium ovale* and for the treatment of Malaria quartana, caused by *Plasmodium malariae*. They are also suitable for the treatment of Toxoplasmosis, caused by *Toxoplasma gondii,* Coccidiosis, caused for instance by *Isospora belli,* intestinal Sarcosporidiosis, caused by *Sarcocystis suihominis,* dysentery caused by *Entamoeba histolytica*, Cryptosporidiosis, caused by *Cryptosporidium parvum*, Chargas' disease, caused by *Trypanosoma cruzi,* sleeping sickness, caused by *Trypanosoma brucei rhodesiense* or *gambiense*, the cutaneous and visceral as well as other forms of Leishmaniosis. They are also suitable for the treatment of animals infected by veterinary pathogenic protozoa, like *Theileria parva*, the pathogen causing bovine East coast fever, *Trypanosoma congolense congolense* or *Trypanosoma vivax vivax, Trypanosoma brucei brucei,* pathogens causing Nagana cattle disease in Africa, *Trypanosoma brucei evansi* causing Surra, *Babesia bigemina*, the pathogen causing Texas fever in cattle and buffalos, *Babesia bovis,* the pathogen causing european bovine Babesiosis as well as Babesiosis in dogs, cats and sheep, *Sarcocystis ovicanis* and *ovifelis* pathogens causing Sarcocystiosis in sheep, cattle and pigs, Cryptosporidia, pathogens causing Cryptosporidioses in cattle and birds, Eimeria and Isospora species, pathogens causing Coccidiosis in rabbits, cattle, sheep, goats, pigs and birds, especially in chickens and turkeys. The use of the compounds of the present invention is preferred in particular for the treatment of Coccidiosis or Malaria infections, or for the preparation of a drug or feed stuff for the treatment of these diseases. This treatment can be prophylactic or curative. In the treatment of malaria, the compounds of the present invention may be combined with other anti-malaria agents.

The compounds of the present invention can further be used for viral infections or other infections caused for instance by *Pneumocystis carinii.*

The compounds of formula (I) or of formula (II), and their pharmacologically acceptable salts can be administered to animals, preferably to mammals, and in particular to humans, dogs and chickens as therapeutics per se, as mixtures with one another or in the form of pharmaceutical preparations which allow enteral or parenteral use and which as active constituent contain an effective dose of at least one compound of the formula (I), or a salt thereof, in addition to customary pharmaceutically innocuous excipients and additives. The compounds of formula (I) or of formula (II), can also be administered in form of their salts, which are obtainable by reacting the respective compounds with physiologically acceptable acids and bases.

The therapeutics can be administered orally, e.g. in the form of pills, tablets, coated tablets, sugar coated tablets, hard and soft gelatin capsules, solutions, syrups, emulsions or suspensions or as aerosol mixtures. Administration, however, can also be carried out rectally, e.g. in the form of suppositories, or parenterally, e.g. in the form of injections or infusions, or percutaneously, e.g. in the form of ointments, creams or tinctures.

In addition to the active compounds of formula (I) or of formula (II), the pharmaceutical composition can contain further customary, usually inert carrier materials or excipients. Thus, the pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain two or more compounds of the formula (I) or of formula (II), or their pharmacologically acceptable salts and also other therapeutically active substances.

Thus, the compounds of the present invention can be used in the form of one substance alone or in combination with other active compounds - for example with medicaments already known for the treatment of the aforementioned diseases, whereby in the latter case a favorable additive, amplifying effect is noticed. Suitable amounts to be administered to humans range from 5 to 500 mg.

To prepare the pharmaceutical preparations, pharmaceutically inert inorganic or organic excipients can be used. To prepare pills, tablets, coated tablets and hard gelatin capsules, for example, lactose, corn starch or derivatives thereof, talc, stearic acid or its salts, etc. can be used. Excipients for soft gelatin capsules and suppositories are, for example, fats, waxes, semi-solid and liquid polyols, natural or hardened oils etc. Suitable excipients for the production of solutions and syrups are, for example, water, sucrose, invert sugar, glucose, polyols etc. Suitable excipients for the production of injection solutions are, for example, water, alcohols, glycerol, polyols or vegetable oils.

The dose can vary within wide limits and is to be suited to the individual conditions in each individual case. For the above uses the appropriate dosage will vary depending on the mode of administration, the particular condition to be treated and the effect desired. In general, however, satisfactory results are achieved at dosage rates of about 1 to 100 mg/kg animal body weight preferably 1 to 50 mg/kg. Suitable dosage rates for larger mammals, for example humans, are of the order of from about 10 mg to 3 g/day, conveniently administered once, in divided doses 2 to 4 times a day, or in sustained release form.

In general, a daily dose of approximately 10 mg to 5000 mg, preferably 50 to 500 mg, per human individual is appropriate in the case of the oral administration which is the preferred form of administration according to the invention. In the case of other administration forms too, the daily dose is in similar ranges.

The compounds of formula (I) or of formula (II), can also be used in the form of a precursor (prodrug) or a suitably modified form, that releases the active *compound in vivo.*

Such precursors such as the preferred embodiments of R⁶ can be obtained for example by masking the free acid group with an ester group, which is then in turn transformed into the free acid group *in vivo* [F. W. Sum et. al. Bioorg. & Med. Chem. Lett. 9 (1999), 1921-1926; Ada Rephaeli et. al. Drug Development Research 50 (2000) 379-391; H. Ishikawa, Current Med. Chem. 6 (1999), 575-597]. Also such precursors for the preferred embodiments of R⁵ can be obtained for example by masking the amidine with an hydroxy group, which is then in turn transformed into the free amidine *in vivo* [R.M. Scarborough, J. Med. Chem. 43, 19, (2000), 3454-3473].

### Examples

### 1. General methode for the synthesis of cyclopentane dicarboxylic acide monoamide

1.0 mmol of cyclopent-1-ene dicarboxylic acid monomamide was suspended under inert atmosphere in 10 ml dry dichloromethane. Platin(IV)oxide (56 µmol, 10mol%) was added and the reaction mixture was stirred for 12 h at room temperature. Platin(IV)oxide was removed by filtration and the filtrate was evaporated in vacuum. The residue was purified by HPLC to give the product. The yield is 50 - 70 % of both isomers.

### 2. General methode for the synthesis of cis and trans isomeres cyclopentane dicarboxylic acide monoamide

1,2 cyclopentane dicarboxylic acid (cis or trans isomere) was suspended in acetic acid andydride and heated to 100°C for 3 h. The reaction solution was cooled to room teinperatur. The solvent was evaporated under reduced pressure and the residue was dried in fine vacuum for 6 h to to give the anhydrid in quantitativ yield. The resulting anhydrid was dissolved in dry dichloromethane (0,36 mmol/ml). To this solution 1 eq of the biphenylamine derivative was added a the reaction mixture was stirred for 12 h. The solvent was removed and the residue was purified by HPLC to give the amide in yields between 40 and 70 % of cis or trans isomere.

### 3. General methode for the synthesis of cyclopent-2-ene dicarboxylic acid monoamide

1.0 mmol of cyclopent-1-ene dicarboxylic acid monoamide was solved under inert atmosphere in 3 ml dry tetrahydrofuran. The mixture was cooled to -78°C and 3.0 mmol n-butyl lithium (1.6mol/l solution) was added slowly. The reaction mixture was stirred for 20 minutes at -78°C and then 16 hours at room temperature. Water was added and the solution was purified by HPLC to give few mg of the product. 5-(3-Fluoro-3'-methoxy-biphenyl-4-ylcarbamoyl)-cyclopent-1-enecarboxylic acid was synthesised by this way. NMR spectra: Bruker Avance 300 MHz. The spectra were recorded in CDCl₃ at 300 MHz (¹H NMR), respectively, using the residual solvent peak as an internal standard (δ= 7.26).

Analytical LC/ESI-MS: 2 x Waters 600 Multisolvent Delivery System. 50µl sample loop. Column, Chromolith Speed ROD RP 18e (Merck, Darmstadt), 50 x 4.6 mm, with 2 µm prefilter (Merck). Eluent A, H₂O+0.1%HCO₂H; eluent B, MeCN. Gradient, 5% B to 100% B within 5 min; flow 3ml/min. Waters LCZ single quadrupol mass spectrometer with electrospray source. MS method, MS8minPM-80-800-20V; positive/negative ion mode scanning, m/z 80 - 800 in 1 s; capillary, 3.5 kV; cone voltage, 20 V; multiplier voltage, 400 V; probe and desolvation gas temperature, 120° C and 350° C, respectively. Waters 2487 Dual λ Absorbance Detector, set to 254 nm.

Example 1: *trans 2-(3-Fluoro-3' methoxy-biphenyl-4-ylcarbamoyl)-cyclopentane carboxylic acid* ¹H-NMR: δ= 1.75 (m_{c}, 2 H, CH₂), 2.02 (m_{c}, 4 H, CH₂), 3.09 (m_{c}, 2 H, CH), 3.79 (s, 3 H, CH₃), 3.84 (s, 3 H, CH₃), 6.79- 8.35 (m, 7 H, CH_{Ar}), 7.87 (s, 1H, NH). LC/(+)-ESI-MS: m/z = 358 [M+H]⁺

Example 2: *cis 2-(3-Fluoro-3' methoxy-biphenyl-4-ylcarbamoyl)-cyclopentane carboxylic acid* ¹H-NMR: *δ=* 1.89 (m_{c} 2 H, CH₂), 2.02 (m_{c}, 4 H, CH₂), 3.25 (m_{c}, 2 H, CH), 3.85 (s, 3 H, CH₃), 3.84 (s, 3 H, CH₃), 6.80- 8.40 (m, 7 H, CH_{Ar}), 7.54 (s,1H, NH). LC/(+)-ESI-MS: m/z = 358 [M+H]⁺

Example 3: *2-(2'-Chloro-3,5-difluoro-biphenyl-4-ylcarbamoyl)-cyclopentane carboxylic acid* ¹H-NMR: δ= 1.82 (m_{c}, 2 H, CH₂), 2.11 (m_{c}, 4 H, CH₂), 3.21 (m_{c}, 2 H, CH), 7,01-7,51 (m, 6 H, CH_{Ar}), 7.41 (s,1H, NH). LC/(+)-ESI-MS: m/z = 380 [M+H]⁺

Example 4: *2-(3,5-Difluoro-2',4'-dimethoxy-biphenyl-4-ylcarbamoyl)-cyclopentane carboxylic acid* ¹H-NMR: δ= 1.80 (m_{c}, 2 H, CH₂), 2.09 (m_{c}, 4 H, CH₂), 3.19 (m_{c}, 2 H, CH), 3.79 (s, 3 H, CH₃), 3.84 (s, 3 H, CH₃), 6.50- 7.22 (m, 5 H, CH_{Ar}), 7.3 (s, 1H, NH). LC/(+)-ESI-MS: m/z = 406 [M+H]⁺

Example 5: *2-(3'-Ethoxy-3,5-difluoro-biphenyl-4-ylcarbamoyl)-cyclopentane carboxylic acid* ¹H-NMR: δ= 1.73 (m_{c}, 3 H, CH₃), 1.81 (m_{c}, 2 H, CH₂), 2.09 (m_{c}, 4 H, CH₂), 3.20 (m_{c}, 2 H, CH), 4.07 (m_{c}, 2 H, CH₂), 6.87- 7.36 (m, 6 H, CH_{Ar}), 7.39 (s,1H, NH). LC/(+)-ESI-MS: m/z = 390 [M+H]⁺

Example 6: *2-(2'-Ethoxy-3,5-difluoro-biphenyl-4-ylcarbamoyl)- cyclopentane carboxylic acid* ¹H-NMR: δ= 1.36 (m_{c}, 3 H, CH₃), 1.81 (m_{c}, 2 H, CH₂), 2.08 (m_{c}, 4 H, CH₂), 3.20 (m_{c}, 2 H, CH), 4.04 (m_{c}, 2 H, CH₂), 6.92- 7.36 (m, 6 H, CH_{Ar}). LC/(+)-ESI-MS: m/z = 390 [M+H]⁺

Example 7: *2-(Biphenyl-4-ylcarbamoyl)-cyclopentane carboxylic acid* ¹H-NMR: δ= 1.81 (m_{c}, 2 H, CH₂), 2.01 (m_{c}, 4 H, CH₂), 3.1 (m_{c}, 2 H, CH), 7.28 - 7.63 (m, 9 H, CH_{Ar}) 7.80 (s,1H, NH). LC/(+)-ESI-MS: m/z = 310 [M+H]⁺

Example 8: *2-(2,3,5,6-Tetrafluoro 3'-trifluoro methoxy-biphenyl-4-ylcarbamoyl)-cyclo-pentane carboxylic acid* ¹H-NMR: δ= 1.82 (m_{c}, 2 H, CH₂), 2.08 (m_{c}, 4 H, CH₂), 3.20 (m_{c}, 2 H, CH), 7.27- 7.71 (m, 4 H, CH_{Ar}). LC/(+)-ESI-MS: m/z = 466 [M+H]⁺

Example 9: *2-(3,5-Difluoro-3'-trifluoro methoxy-biphenyl-4-ylcarbamoyl)-cyclopentane carboxylic acid* ¹H-NMR: δ= 1.83 (m_{c}, 2 H, CH₂), 2.10 (m_{c}, 4 H, CH₂), 3.20 (m_{c}, 2 H, CH), 7.11- 7.50 (m, 6 H, CH_{Ar}). LC/(+)-ESI-MS: m/z = 430 [M+H]⁺

### 4. Inhibition Assay of DHODH activity

The standard assay mixture contained 50 µM decyclo ubichinone, 100 µM dihydroorotate, 60 µM 2,6-dichloroindophenol, as well as 20 mU DHODH. The volume activity of the recombinant enzyme used was 30 U/ml. Measurements were conducted in 50 mM TrisHCl (150 mM KCl, 0,1% Triton X-100, pH 8,0) at 30°C in a final volume of 1 ml. The components were mixed, and the reaction was started by adding dihydroorotate. The course of reaction was followed by spectrophotometrically measuring the decrease in absorption at 600 nm for 2 min.
Inhibitory studies were conducted in a standard assay with additional variable amounts of inhibitor. For the determination of the IC₅₀ values (concentration of inhibitor required for 50% inhibition) at least five different inhibitor concentrations were applied.
These investigations were carried out with recombinant human as well as with recombinant murine DHODH provided by Prof. M. Löffler, Marburg, Germany [M. Löffler, Chem. Biol. Interact. 124, (2000), 61-76].

Example 2, 3, 5, 7, 8 and 9 showed IC₅₀-values (human DHODH) of < 5 µM. Example 1, 4, and 6 showed IC₅₀-values (human DHODH) between 5 µM and 20 µM..

## Claims

1. A compound of the general formula (I) 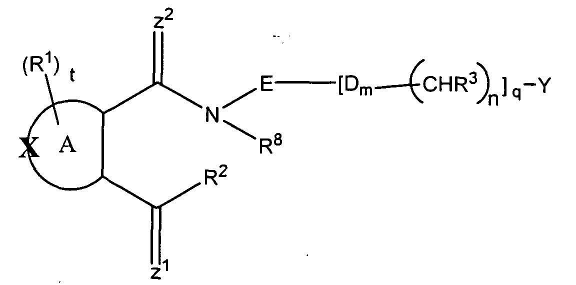 or salts or isomeres thereof, wherein
A is a 4-8 membered non-aromatic ring system, wherein one or more of the carbon atoms in the ring can be replaced by a group X, wherein X is selected from the group consisting of S, O, N, NR⁴, SO, CO or SO₂;
D is O, S, SO₂, NR⁴ or CH₂;
Z¹ and Z² are independent from each other O, S, or NR⁵ ;
R¹ independently represents H, halogen, haloalkyl, haloalkyloxy -CO₂R'',--SO₃H, -OH, -CONR*R'', -CR''O, -SO₂-NR*R'', -NO₂, -SO₂-R'', -SO-R*, -CN, alkoxy, alkylthio, aryl, -NR''-CO₂-R', -NR''-CO-R*, -NR''-SO₂-R', -O-CO-R*, -NR*R'', -NR*OR''-O-CO₂-R*, -O-CO-NR*R''; cycloalkyl, alkylamino, hydroxyalkylamino, -SH, heteroaryl, or alkyl;
R* independently represents H, alkyl, cycloalkyl, aminoalkyl, alkoxy, -OH, -SH, alkylthio, hydroxyalkyl, haloalkyl, haloalkyloxy, aryl or heteroaryl;
R' independently represents H, -CO₂R'', -CONHR", -CR''O, -SO₂NR'', -NR"-CO-haloalkyl, -NO₂, -NR''-SO₂-haloalkyl, -NR''-SO₂-alkyl, -SO₂-alkyl, -NR"-CO-alkyl, -CN, alkyl, cycloalkyl, aminoalkyl, alkylamino, alkoxy, -OH, -SH, -NR''R*, -NR''OR*, alkylthio, hydroxyalkyl, hydroxyalkylamino, halogen, haloalkyl, haloalkyloxy, aryl, arylalkyl or heteroaryl;
R" independently represents hydrogen, haloalkyl, hydroxyalkyl, alkyl, cycloalkyl, aryl, heteroaryl or aminoalkyl;
R² is H, OR⁶, NHR⁷, or R² togehter with the nitrogen atom to which R⁸ is attached forms a 5 or 6 membered heterocyclic ring with the proviso that R² is -[CH₂]₀₋₃ and R⁸ is absent;
R³ is H, alkyl, cycloalkyl, aryl, arylalkyl, alkoxy, O-aryl; O-cycloalkyl, halogen, aminoalkyl, alkylamino, hydroxylamino, hydroxylalkyl, haloalkyl, haloalkyloxy, heteroaryl, alkylthio, S-aryl, or S-cycloalkyl;
R⁴ is H, alkyl, cycloalkyl, aryl, or heteroaryl;
R⁵ is H, OH, alkoxy, O-aryl, alkyl, or aryl;
R⁶ is H, alkyl, cycloalkyl, aryl, heteroaryl, arylalkyl, alkylaryl, alkoxyalkyl, acylmethyl, (acyloxy)alkyl, non-symmetrical (acyloxy)alkyldiester, or dialkylphosphate;
R⁷ is H, alkyl, aryl, alkoxy, O-aryl, cycloalkyl, or O-cycloalkyl;
R⁸ is hydrogen or alkyl;
E is an alkyl or cycloalkyl group or a monocyclic or polycyclic substituted or unsubstituted ring system which may contain one or more groups X and which contains at least one aromatic ring;
Y is hydrogen, halogen, haloalkyl, haloalkyloxy, alkyl, cycloalkyl, a monocyclic or polycyclic substituted or unsubstituted ring system which may contain one or more groups X and which contains at least one aromatic ring or 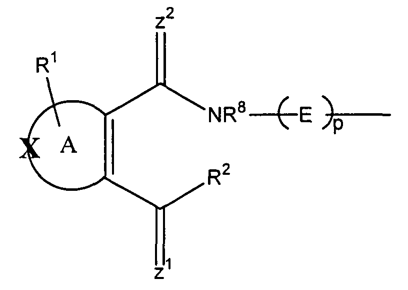
m is 0 or 1;
n is 0 or 1;
p is 0 or 1;
q is 0 or 1;
t is 1 to 3;
With the proviso that trans-2-[4-(Naphthalin-2-yl)thiazol-2-ylaminocarbonyl]cyclopentane carboxylic acid is excluded.

2. A compound of the general formula (II) 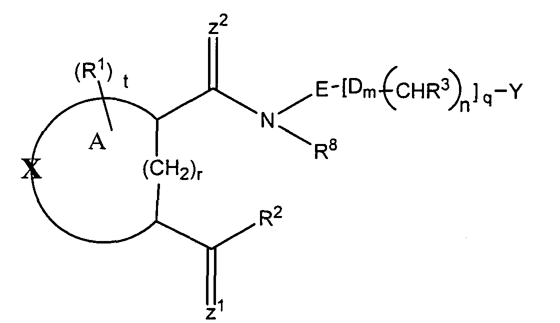 or salts or isomeres thereof, wherein
A is a 3-8 membered non-aromatic ring system, wherein the ring system comprises at least one double bond and wherein one or more of the carbon atoms in the ring can be replaced by a group X, wherein X is selected from the group consisting of S, O, N, NR⁴, SO, CO or SO₂, wherein, when r = 0, there is no double bond between the carbon atoms carrying the substituents
-CZ¹- and -CZ²-;
D is O, S, SO₂, NR⁴ or CH₂;
Z¹ and Z² are independent from each other O, S, or NR⁵ ;
R' independently represents H, halogen, haloalkyl, haloalkyloxy -CO₂R'', -SO₃H, -OH, -CONR*R'', -CR''O, -SO₂-NR*R'', -NO₂, -SO₂-R'', -SO-R*, -CN, alkoxy, alkylthio, aryl, -NR''-CO₂-R', -NR''-CO-R*, -NR''-SO₂-R', -O-CO-R*, -NR*R'', -NR*OR''-O-CO₂-R*, -O-CO-NR*R''; cycloalkyl, alkylamino, hydroxyalkylamino, -SH, heteroaryl, or alkyl;
R* independently represents H, alkyl, cycloalkyl, aminoalkyl, alkoxy, -OH, -SH, alkylthio, hydroxyalkyl, haloalkyl, haloalkyloxy, aryl or heteroaryl;
R' independently represents H, -CO₂R'', -CONHR", -CR''O, -SO₂NR'', -NR"-CO-haloalkyl, -NO₂, -NR''-SO₂-haloalkyl, -NR''-SO₂-alkyl, -SO₂-alkyl, -NR"-CO-alkyl, -CN, alkyl, cycloalkyl, aminoalkyl, alkylamino, alkoxy, -OH, -SH, -NR''R*, -NR''OR*, alkylthio, hydroxyalkyl, hydroxyalkylamino, halogen, haloalkyl, haloalkyloxy, aryl, arylalkyl or heteroaryl;
R" independently represents hydrogen, haloalkyl, hydroxyalkyl, alkyl, cycloalkyl, aryl, heteroaryl or aminoalkyl;
R² is H, OR⁶, NHR⁷, NHOR⁶ or R² togehter with the nitrogen atom to which R⁸ is attached forms a 5 or 6 membered heterocyclic ring with the proviso that R² is -[CH₂]₀₋₃ and R⁸ is absent;
R³ is H, alkyl, cycloalkyl, aryl, arylalkyl, alkoxy, O-aryl; O-cycloalkyl, halogen, aminoalkyl, alkylamino, hydroxylamino, hydroxylalkyl, haloalkyl, haloalkyloxy, heteroaryl, alkylthio, S-aryl, or S-cycloalkyl;
R⁴ is H, alkyl, cycloalkyl, aryl, or heteroaryl;
R⁵ is H, OH, alkoxy, O-aryl, alkyl, or aryl;
R⁶ is H, alkyl, cycloalkyl, aryl, heteroaryl, arylalkyl, alkylaryl, alkoxyalkyl, acylmethyl, (acyloxy)alkyl, non-symmetrical (acyloxy)alkyldiester, or dialkylphosphate;
R⁷ is H, alkyl, aryl, alkoxy, O-aryl, cycloalkyl, or O-cycloalkyl;
R⁸ is hydrogen or alkyl;
E is an alkyl or cycloalkyl group or a monocyclic or polycyclic substituted or unsubstituted ring system which may contain one or more groups X and which contains at least one aromatic ring;
Y is hydrogen, halogen, haloalkyl, haloalkyloxy, alkyl, cycloalkyl, a monocyclic or polycyclic substituted or unsubstituted ring system which may contain one or more groups X and which contains at least one aromatic ring or 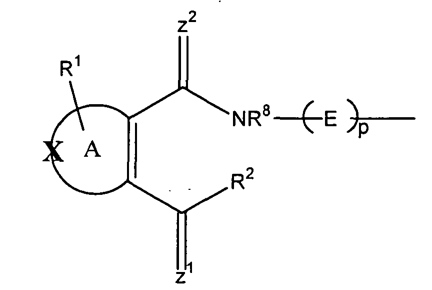
m is 0 or 1;
n is 0 or 1;
p is 0 or 1;
r is 0 or 1; and
q is 0 or1;
t is 1 to 3;

3. The compound according to claim 1 or 2, wherein R¹ is selected from the group consisting of OH, CO₂H and SO₃H.

4. The compound according to any one of the preceding claims, wherein both Z' and Z² are O.

5. The compound according to any one of the preceding claims, wherein E is selected from the group consisting of phenyl, 1-naphtyl, 2-naphthyl, 1-anthracyl and 2-anthracyl and is optionally substituted with one or more substituents R'.

6. The compound according to claim 1, wherein q=0, t=1, A is a carbocyclic non-aromatic ring system, Y is H or F, and E is phenyl which is optionally substituted with at least one substituent selected from the group consisting of Cl, F, CF₃, OCF₃, O-methyl and O-ethyl.

7. The compound according to claim 1, wherein q=0, t=1, A is a carbocyclic non-aromatic ring system, and E and Y are phenylene and phenyl, respectively, wherein E is optionally substituted with at least one substituent selected from the group consisting of Cl and F and Y is optionally substituted with at least one substituent selected from the group consisting of O-methyl, O-ethyl, OCF₃, Cl and F.

8. The compound according to claim 7, wherein Y and -NCR⁸ are in para position on E.

9. A compound according to any one of claims 1 to 8 for the use as a medicament.

10. A pharmaceutical composition comprising a compound as defined in any one of claims 1 to 9 in free form or in the form of pharmaceutically acceptable salts and physiologically functional derivatives, together with pharmaceutically acceptable diluents or carriers.

11. The use of a compound of according to any one of claims 1 to 9 and physiologically functional derivatives and their pharmacologically tolerable salts in the manufacture of a medicament for use in treatment of a disease or a therapeutic indication in which inhibition of dihydrooratate dehydrogenase is beneficial.

12. The use of claim 11 wherein the disease or indication is selected from the group consisting of rheumatism, acute immunological disorders, autoimmune diseases, diseases caused by malignant cell proliferation, inflammatory diseases, diseases that are caused by protozoa infestations in humans and animals, diseases that are caused by viral infections or *Pneumocystis carinii*, fibrosis, uveitis, rhinitis, asthma or athropathy.

13. The use of a compound as defined in any one of claims 1 to 8 for the inhibition of DHODH.
